# EUROPEAN PATENT APPLICATION

(11) **EP 4 661 020 A1**
(43) Date of publication of application: **10.12.2025**
(21) Application number: 25179651.2
(22) Date of filing: 29.05.2025
(51) Int. Cl.: G16H 20/40, G16H 40/63, G16H 40/67, G16H 50/30, A61N 5/10

(54) **USING MACHINE-LEARNING LANGUAGE PROCESSING MODEL FOR RADIOTHERAPY VISUALIZATION**

(30) Priority: 03.06.2024 US 202418732529
(71) Applicant: Siemens Healthineers International AG, 6312 Steinhausen (CH)
(72) Inventor: PELTOLA, Jarkko, 04300 Tuusula (FI); KUUSELA, Esa, 02320 Espoo (FI); HAUTALA, Ismo, 02600 Espoo (FI)
(74) Representative: Mathisen & Macara LLP

(57) **Abstract**

Disclosed herein are methods (200) and systems (100) for generating visualizations of radiation therapy treatments utilizing machine learning. One method (200) involves presenting (202) a user interface on a user device, designed for medical professionals, comprising an interaction interface. Through this interface, the processor receives (204) one or more visualization attributes associated with a patient's radiation therapy treatment. Subsequently, a machine-learning model is executed (206) by the processor, utilizing the received visualization attributes to generate machine-readable code. This code instructs a visualization software module to generate a visualization corresponding to the provided attributes. Upon generation, the machine-readable code is transmitted (208) to the software module, which responds by presenting (210) the visualization via the user interface. This method enhances the efficiency and accuracy of visualizing radiation therapy treatments, facilitating informed decision-making by medical professionals.

## Description

### TECHNICAL FIELD

This application relates generally to generating a radiotherapy treatment plan visualization using a machine learning language processing model.

### BACKGROUND

Radiotherapy or radiation therapy (RT) is one of the main modalities used in cancer treatment, and RT treatment planning (RTTP) is a complex process that contains specific guidelines, protocols, and instructions adopted by different medical professionals, such as clinicians, medical device manufacturers, and the like. Medical professionals strive to deliver the safest and most effective treatment to patients.

The RTTP creation process typically involves a collaboration of multiple professionals and an automated computer model (e.g., treatment planner). The initial treatment plan is usually created based on the best available information at the time, tailored from standard treatment protocols to suit the individual patient. The medical professionals review various attributes of a treatment plan and may perform multiple rounds of additional interaction with the computer model before approving the plan. One of the most common ways of reviewing attributes of a treatment plan is using visualization of the treatment and how it would/could affect the patients (e.g., dose volume histograms, color-coded contour maps, and/or interactive treatment simulations). Therefore, visualizing a radiotherapy plan effectively can significantly enhance the planning of, understanding, and execution of treatment. These visualization methods provide critical insights that help improve the accuracy and effectiveness of radiotherapy treatments, ultimately leading to better patient outcomes.

In order to visualize and review the treatment plan (or the potential treatment plan), medical professionals are constrained by the capabilities of their existing software (e.g., visualization software). Some medical professionals find it unattainable to request additional information-often in a visual format-regarding a particular aspect of the treatment plan. Overall, the power of visualization of a treatment plan is also constrained by the medical professional's ability to operate their visualization software, which is highly undesirable because it is highly dependent upon the medical professional's individual skills. In one example, visualizing three-dimensional dose distributions is a significant challenge for some medical professionals. The intricacies of this process require precise visual tools that help medical professionals understand how radiation doses interact with various organs.

Current software solutions, however, are often cumbersome, featuring outdated interfaces and limited customization options. This technical shortcoming can hinder the ability to make informed decisions quickly and adapt treatments to patient-specific needs, ultimately impacting the effectiveness and efficiency of the RTTP.

Generally, integrating new visual features into treatment planning software involves prolonged discussions between customers and software providers, which can take a long time to result in an actual product update. In some instances, users (e.g., a particular medical professional or a clinic) develop their own software solutions to visualize specific aspects of the treatment. However, they are still limited by the data they can extract from the treatment planning software or computer models.

### SUMMARY

In accordance with a first aspect of the invention, there is provided a method as defined by claim 1.

In accordance with a second aspect of the invention, there is provided a system as defined by claim 8.

Optional features are defined by the dependent claims.

Embodiments disclosed herein include computing systems that execute software components for machine-learning architecture functions, including an artificial intelligent visualization assistant ("AI assistant" or "AI agent"), to improve treatment plan visualizations to improve the efficiency and efficacy of treatment planning. Using the methods and systems discussed herein, a server may employ various AI tools and techniques to generate real-time or near-real-time data visualizations tailored to user needs at any stage of the treatment process, or any stage of the treatment planning process.

In one embodiment, a method comprises presenting, by a processor on a user device, a user interface comprising an interaction interface for a medical professional operating the user device; receiving, by the processor from the interaction interface, one or more visualization attributes associated with a radiation therapy treatment of a patient; executing, by the processor, a machine-learning language processing model using the one or more visualization attributes to generate machine readable code instructing a visualization software module to generate a visualization corresponding to the one or more visualization attributes; transmitting, by the processor, the machine-readable code, to the software module; and in response to receiving the visualization from the visualization software module, presenting, by the processor, the visualization via the user interface.

The machine-readable code may comprise or be included in a digital library to generate the visualization.

The machine-learning language processing model may be specifically trained for a plan optimizer that generates the radiation therapy treatment of the patient.

The method may further comprise recalibrating, by the processor, the machine-learning language processing model using an input corresponding to a quality of the visualization.

The method may further comprise adjusting, by the processor, an attribute of the treatment plan in accordance with an input received after presenting the visualization.

The visualization may be a comparison between an attribute of the treatment plan at two different times, or at two different stages of the planning process.

In another embodiment, a system may comprise a non-transitory computer readable medium having instructions that when executed, cause a processor to: present, on a user device, a user interface comprising an interaction interface for a medical professional operating the user device; receive, from the interaction interface, one or more visualization attributes associated with a radiation therapy treatment of a patient; execute a machine-learning language processing model using the one or more visualization attributes to generate machine readable code instructing a visualization software module to generate a visualization corresponding to the one or more visualization attributes; transmit the machine-readable code, to the software module; and in response to receiving the visualization from the visualization software module, present the visualization via the user interface.

The machine-readable code may comprise or be included in a digital library to generate the visualization.

The machine-learning language processing model may be specifically trained for a plan optimizer that generates the radiation therapy treatment of the patient.

The machine-learning language processing model may be specifically trained for the visualization using at least one configuration of the visualization engine.

The instructions may further cause the processor to recalibrate the machine-learning language processing model using an input corresponding to a quality of the visualization.

The instructions may further cause the processor to adjust an attribute of the treatment plan in accordance with an input received after presenting the visualization.

The visualization may be a comparison between an attribute of the treatment plan at two different times, or at two different stages of the planning process.

In another embodiment, a system comprises a machine-learning language processing model; and a processor in communication with the machine-learning language processing model, the processor configured to: present, on a user device, a user interface comprising an interaction interface for a medical professional operating the user device; receive, from the interaction interface, one or more visualization attributes associated with a radiation therapy treatment of a patient; execute the machine-learning language processing model using the one or more visualization attributes to generate machine readable code instructing a visualization software module to generate a visualization corresponding to the one or more visualization attributes; transmit the machine-readable code, to the software module; and in response to receiving the visualization from the visualization software module, present the visualization via the user interface.

The machine-readable code may comprise or be included in a digital library to generate the visualization.

The machine-learning language processing model may be specifically trained for a plan optimizer that generates the radiation therapy treatment of the patient.

The machine-learning language processing model may be specifically trained for the visualization using at least one configuration of the visualization engine.

The processor may be further configured to recalibrate the machine-learning language processing model using an input corresponding to a quality of the visualization.

The visualization may be a comparison between an attribute of the treatment plan at two different times, or at two different stages of the planning process.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a system implementing an AI visualization agent, according to an embodiment.
**FIG. 2** shows an operational workflow of a method performed in hardware and software computing components that host and execute an AI visualization agent, in accordance with an embodiment.

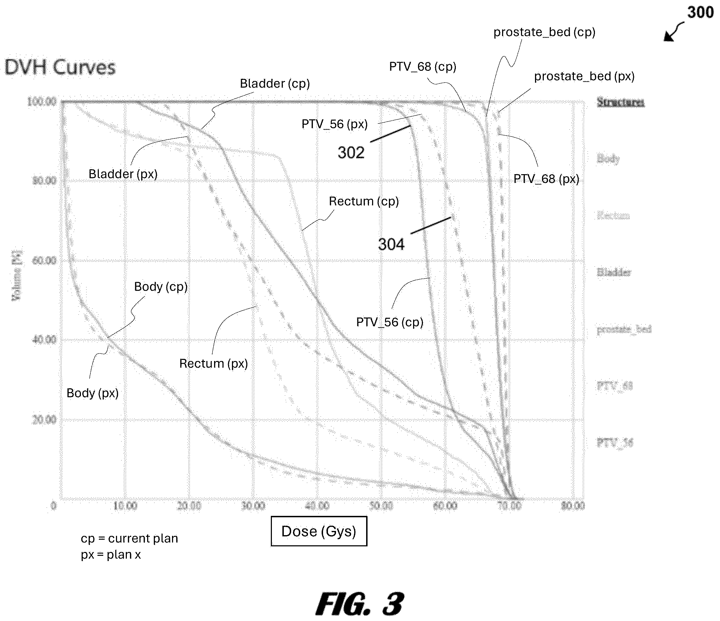

**FIG. 3** illustrates a non-limiting example of a visualization generated in an AI visualization agent, in accordance with an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used, and/or other changes may be made without departing from the scope of the present disclosure. The illustrative embodiments described in the detailed description are not meant to limit the subject matter presented.

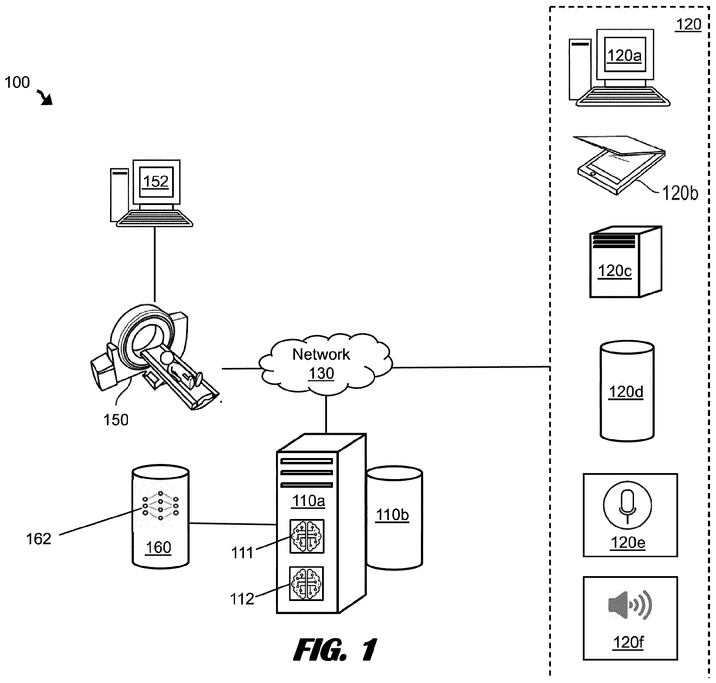

**FIG. 1** illustrates components of a system **100** for an AI visualization agent, according to an embodiment. The system **100** may include an analytics server **110a**, a system database **110b**, a machine learning language processing model **111** for visualizing treatment plan data, end-user devices **120a-120f** (collectively end-user devices **120**), a medical device **150**, a medical device computer **152**, a database **160**, and a radiotherapy plan optimizer **162**. Various components depicted in **FIG. 1** may belong to a radiation therapy treatment clinic at which patients may receive radiation therapy treatment, in some cases via one or more radiation therapy machines (e.g., the medical device **150**).

The system **100** is not confined to the components described herein and may include additional or other components, not shown for brevity, which are to be considered within the scope of the embodiments described herein.

The above-mentioned components may be connected to each other through one or more networks **130**. Examples of the network **130** may include, but are not limited to, private or public local-area networks (LAN), wireless local-area networks (WLAN), metropolitan-area networks (MAN), wide-area networks (WAN), and the Internet. The network **130** may include wired and/or wireless communications according to one or more standards and/or via one or more transport mediums. The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), or EDGE (Enhanced Data for Global Evolution) network.

The analytics server **110a** may generate and display an electronic platform configured to interface a user with the machine learning language processing model **111** and for receiving patient information (via various sources) and visualization preferences/instructions. The analytics server **110a** may then output the results of the execution of the machine learning language processing model **111** (generated via the visualization engine **112**) and the radiotherapy plan optimizer **162**. The electronic platform may include graphical user interfaces (GUI) displayed on one or more of, or each of, the end-user devices **120**, the medical device **150**, and/or the medical device computer **152**. An example of the electronic platform generated and hosted by the analytics server **110a** may be a web-based application or a website configured to be displayed on different electronic devices, such as mobile devices, tablets, personal computers, and the like.

The platform hosted on the analytics server **110a** or another device of the system **100** includes collaboration software accessible to the user devices **120** of the participating members, such that multiple medical professionals can collaborate and view the visualizations provided by the analytics server **110a**. The collaboration software may include any type of software facilitating user-group collaborations, which may include live interaction software (e.g., teleconferencing software) or asynchronous collaborations (e.g., online postings). Non-limiting examples of collaboration software include MS Teams^{®}, Skype^{®}, WebEx^{®}, Slack^{®}, and Twilio^{®}, among others. The collaboration software may also facilitate communication between one or more medical professionals and the analytics server **110a**. For instance, the platform provided or hosted by the analytics server **110a** may include an input element (e.g., visual, textual, or auditory), allowing users (e.g., one or more medical professionals) to input their desired visualization attributes.

The analytics server **110a** may use one or more software module components (e.g., plug-in) of the collaboration software of the platform of the analytics server **110a**.

The information displayed by the analytics server **110a** of the electronic platform can include, for example, input elements to receive data associated with a patient to be treated (e.g., plan objectives) or visualization attributes (e.g., what the medical professional desires to view) and display results as facilitated by the machine learning language processing model **111**, which may include various formats of responsive predicted outputs (e.g., text, images, or videos generated in response to inputs received through the TBA or electronic platform). Specifically, the outputs produced by the machine learning language processing model **111** or the analytics server **110a** may be fed to the radiotherapy plan optimizer **162** (e.g., a predicted radiotherapy plan) and the visualization engine **112**. For instance, the analytics server **110a** may transmit the input received via the user device **120** to the machine learning language processing model **111**. The machine learning language processing model **111** may then generate code that can be transmitted (via the analytics server **110a**) to the visualization engine **112**. The generated code may be based on the transmitted input. The visualization engine may then generate the required visualization whereby the analytics server then displays the results for the participants of the platform at a user device **120** and/or another medical professional at the medical device **150**. The generated visualization may be based on the transmitted code. In some embodiments, the medical device **150** can be a diagnostic imaging device or a treatment delivery device.

The analytics server **110a** may be any computing device comprising a processor and non-transitory machine-readable storage capable of executing the various tasks and processes described herein. The analytics server **110a** may employ various processors, such as central processing units (CPU) and graphics processing units (GPU), among others. Non-limiting examples of such computing devices may include workstation computers, laptop computers, server computers, and the like. While the system **100** includes a single analytics server **110a**, the analytics server **110a** may include any number of computing devices operating in a distributed computing environment, such as a cloud environment.

End-user devices **120** may be any computing device comprising a processor and a non-transitory machine-readable storage medium capable of performing the various tasks and processes described herein. Non-limiting examples of an end-user device **120** may be a workstation computer, laptop computer, tablet computer, or server computer. In operation, various users may use end-user devices **120** to access the GUI operationally managed by the analytics server **110a**. Specifically, the end-user devices **120** may include a clinic computer **120a,** a clinic server **120b**, and medical professional devices **120c**, which may include any electronic devices operated by members of the Tumor Board, medical professionals, and scientists that access and review various types of patient-related treatment data and RTTPs for the patient, among other types of data and information exchanges.

In a non-limiting example, multiple medical professionals may operate the medical professional devices **120c** to review patient-related treatment data to develop a consensus on a treatment for the patient. Even though referred to herein as "end-user" devices, these devices may not always be operated by end-users. For instance, the clinic server **120b** may not be directly used by an end-user. However, the results stored on the clinic server **120b** may be used to populate various GUIs accessed by an end-user via the medical professional device **120c**. Patient-related information generated by the various types of devices of the system **100**, outside the context of the AI visualization agent may be stored within the database **100b**. The stored patient data may be referenced by the analytics server **110a** for training the machine learning language processing model **111**.

The medical device **150** may be a radiation therapy machine configured to implement a patient's radiotherapy treatment. The medical device **150** may also be in communication with a medical device computer **152** that is configured to display various GUIs discussed herein. For instance, the analytics server **110a** may display the results predicted by the radiotherapy plan optimizer **162** onto the computing devices described herein.

The machine learning language processing model **111** may be stored in the system database **110b**. The machine learning language processing model **111** may be configured or trained to automatically generate text, image, or video responses based on inputs received at a user interface or other types of inputs (e.g., speech, such as speech captured at a conference room microphone or microphone of an end-user device **120**).

The user-provided inputs may include text inputs and/or audio signals, for example, text inputs entered by one or more medical professionals or audio signals containing speech audio of the medical professional captured by microphones of the user devices **120** or conference room. The analytics server **110a** may include or invoke Automated Speech Recognition (ASR) software that converts speech audio to text, such as written text. The ASR software comprises a machine-learning architecture trained to detect portions or frames of the audio signal containing the speech audio, such as speech audio of a member speaker. The ASR also comprises and applies Natural Language Processing (NLP) layers of the machine-learning architecture that generates text-based output from the portions of the audio signal containing the detected speech audio. The text generated by the ASR may be fed as an input to the machine learning language processing model **111**. In some embodiments, the ASR may be incorporated within (e.g., be a feature of) the machine learning language processing model **111**.

The machine learning language processing model **111** may be trained to generate machine-readable instructions that can be transmitted to a visualization engine **112** and/or the radiotherapy plan optimizer **162**. The visualization engine **112** may be a collection of software and computer model(s) trained to generate visualizations representing a treatment plan using data generated by the radiotherapy plan optimizer **162**. Additionally, or alternatively, the visualization may be performed by the machine learning language processing model **111** itself.

In some embodiments, the analytics server **110a** may execute the radiotherapy plan optimizer **162** to generate one or more treatment attributes for an RTTP complying with any radiation therapy plan objectives based on patient attributes of a patient for which the radiotherapy treatment plan is being generated. The radiotherapy plan optimizer **162** can be stored in the database **160**. The radiotherapy plan optimizer **162** can generate the one or more treatment attributes, for example, by iteratively calculating the one or more treatment attributes where, with each iteration, the radiotherapy plan optimizer **162** can revise the one or more treatment attributes of the RTTP in accordance with a cost value. This may comprise optimizing a cost function.

The analytics server **110a** may deploy the radiotherapy plan optimizer **162** to generate an RTTP for a patient based on patient attributes. The radiotherapy plan optimizer **162** may iteratively calculate one or more treatment attributes of the RTTP. For instance, with each iteration, the radiotherapy plan optimizer **162** may generate a candidate RTTP having various attributes. The plan optimizer **162** may then use one or more loss functions to calculate a cost value for the generated candidate RTTP. The cost value may indicate a likelihood of the candidate RTTP violating a set of rules, whether internal and/or external rules. For instance, the cost value may indicate whether the candidate RTTP violates any of the plan objectives. The radiotherapy plan optimizer **162** may analyze the cost value. If needed (e.g., when the cost value satisfies a threshold), the radiotherapy plan optimizer **162** may revise the candidate RTTP and re-execute its loss function to generate a new cost value.

Depending on whether the new cost function is increasing or decreasing, the plan optimizer computer model may revise the candidate RTTP again and recalculate the cost value. The radiotherapy plan optimizer **162** may continue this iterative approach until converging upon an RTTP (or the final RTTP) that has a cost value that satisfies a threshold. In some implementations, the treatment attribute for the patient may also indicate how the radiotherapy treatment may be combined or sequentially implemented with other types of treatment modalities (e.g., surgery, chemotherapy).

The analytics server **110a** can identify the treatment attributes that the radiotherapy plan optimizer **162** determined have a cost value that satisfies the threshold. The analytics server **110a** can present the treatment attributes as an RTTP at the end-user device **120** being accessed by the user, generating the radiotherapy treatment plan. Specifically, the analytics server **110a** may utilize the visualization engine **112** to visualize various attributes of the treatment plan.

In some embodiments, the analytics server **110a** or the end-user device **120** can use the RTTP to automatically control the medical device **150** based on attributes of the RTTP to treat the patient.

The system database **110b** may contain data used to train the machine learning language processing model **111.** For instance, the system database **110b** may include data associated with previously treated patients, such as one or more of patient diagnosis data (e.g., tumor data or tumor location), biometric data (e.g., BMI, body weight, height, or various other bodily measurements), and the like. Additionally, the system database **110b** may include visualizations corresponding to one or more of the previously treated patients as well. Therefore, the system database **110b** may include some or all data associated with how the previously treated patients were diagnosed and treated and/or how the treatment plan was visualized. As described herein, the analytics server **110a** may use the data stored within the system database **110b** to train the machine learning language processing model **111.** The analytics server **110a** may also use the data stored within the database **110b** to visualize a particular patient's visualization itself.

The database **110b** may also include various visualization libraries used by the analytics server **110a** and/or the visualization engine **112** to generate the visualizations. In a non-limiting example, a digital library may be a collection of modules and packages that provide pre-written code to help the visualization engine **112** generate the requested visualizations. These libraries can include functions, classes, and variables that are organized in a way that makes them easy to integrate into the visualization engine. The visualization engine **112** may use the pre-written code to generate the requested visualizations.

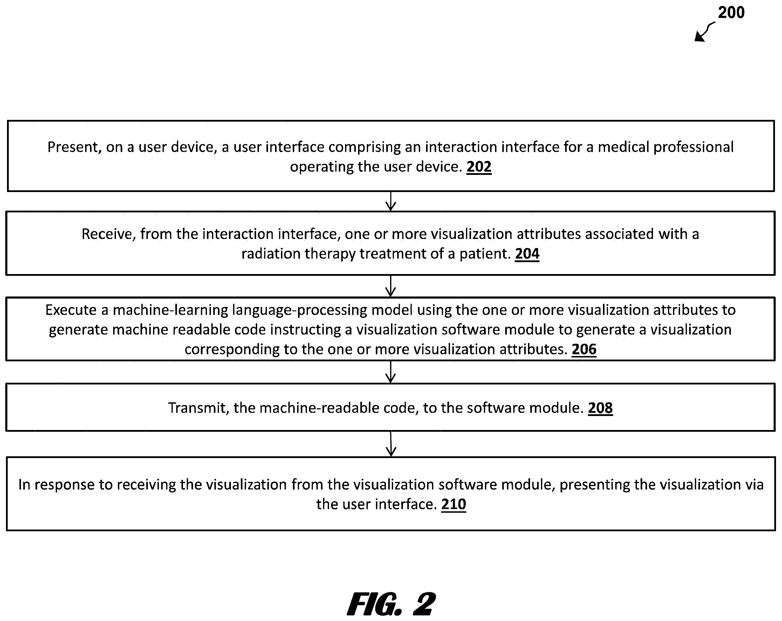

**FIG. 2** shows an operational workflow of method **200** performed in hardware and software computing components that host an AI visualization agent in accordance with an embodiment. The method **200** may include steps **202-210.** However, other embodiments may include additional or alternative steps or may omit one or more steps altogether. The method **200** is described as being executed by a server, such as the analytics server described in **FIG. 1**. However, one or more steps of the method **200** may be executed by any number of computing devices operating in the distributed computing system described in **FIG. 1**. For instance, one or more computing devices may locally perform some or all of the steps described in **FIG. 2**.

Using the method **200**, medical professionals having little or no coding experience can work with different visualization engines and/or plan optimizer computer models, such that they generate customized visualizations. The method **200** allows for the creation of simple, script-like code that is robust and supported by a plethora of radiotherapy software applications. The code or machine-readable script can be customized for different visualization engines and/or different plan optimizers. For instance, a particular clinic can tune the machine learning language processing model in accordance with their clinic's software/version of software applications. In this way, the machine-readable script can instruct various visualization engines (e.g., different software). For instance, the machine learning model discussed herein can be tuned such that the method **200** is used to retrofit existing computing infrastructure.

Accordingly, the machine learning language processing model may be specifically trained to predict/generate machine-readable instructions that are configured for a particular visualization engine. For instance, the training dataset used to train the machine learning language processing model can be limited to a particular visualization engine, such that the results are tailored for that particular visualization engine.

Using the method **200**, the analytics server can generate scripts that produce standard visualizations, such as drawing 2D graphs or visualizing 3D surfaces, which are typical textbook representations of data within the treatment plan. This feature is particularly useful in treatment planning, where the essential data is often in standard 2D or 3D formats, and the required visualizations primarily involve direct algebraic representations of data calculations. The method **200** enables medical professionals to utilize a large language model to (indirectly) generate visualizations of the desired data at any stage of the treatment planning workflow. The code or script for these visualizations may be produced directly by the large language model rather than relying on pre-installed, manually created software that is often static and/or requires coding experience to operate.

At step **202**, the analytics server may present, on a user device, a user interface comprising an interaction interface for a medical professional operating the user device.

The analytics server may present a user interface on a computing device operated by a medical professional (e.g., end-user device **120** discussed in **FIG. 1**). The analytics server may host a platform having various user interfaces that include collaboration software. The analytics server may then transmit the user interface to the computing device over a network. The analytics server may generate and transmit the user interface to the computing device when providing an electronic platform that facilitates discussions regarding treatment considerations and planning (e.g., RTTP).

The analytics server can generate the user interface to have an interaction interface. The interaction interface can be an interface that enables communication between a user (medical professional) viewing and providing inputs into an interaction interface and the analytics server or a machine learning language processing model. The machine learning language processing model can be a large language model that has been trained to generate machine-readable code or instructions, as discussed below with respect to the step **206**. The machine learning language processing model can be or include a neural network, such as a neural network with a transformer architecture.

The interaction interface can enable a form of communication similar to a conversation between two humans that can involve back-and-forth exchanges of messages. In this case, the participants may be expert users who are medical professionals, such as an oncologist or other clinician, and an AI agent that implements the machine learning language processing model to ingest inputs from the discussion and provide the outputs of the machine learning language processing model (e.g., the machine-readable code).

In some implementations, the interaction interface can simulate an instant messaging application conversation between the medical professional(s) and the machine learning language processing model. For example, a user can input text into the interaction interface. The interaction interface can be a feed (e.g., a user feed). The text can be or include patient attributes for a patient receiving radiotherapy treatment. Moreover, the text can be requested for visualizations that are related to a patient's treatment plan (or proposed treatment plan). In a non-limiting example, the input may be "I would like to see a DVH for John Smith, but highlight the OARs in blue."

As discussed herein, the medical professional can use a variety of methods to interact with the interaction interface, such as any combination of one or more of text, audio, video, and the like.

In some embodiments, the interaction interface can cover a portion of the user interface or be a widget of the user interface. The user interface can include other portions or widgets that offer differing functionality, such as external software tools. While the interaction interface can enable communication between the medical professional and the AI agent(s) associated with machine learning language processing model(s), the analytics server can also include a portion of the user interface that displays, for example, different patient attributes of patients, treatment options, or other types of outputs. For example, the analytics server may capture an audio signal in which a medical professional mentions a particular patient attribute, treatment option, and/or visualization attribute. The analytics server may then convert the audio signal or speech signal into a text input (or other input) for the AI agent.

Referring again to **FIG. 2**, at step **204**, the analytics server may receive, from the interaction interface, one or more visualization attributes associated with a radiation therapy treatment of a patient. The medical professional may use a variety of methods (e.g., text, speech or audio) to provide one or more visualization attributes. As used herein, a visualization attribute may be any attribute that describes the visualization desired or inputted by the medical professional. In some embodiments, the visualization attribute may also include or co-occur with patient attributes.

The machine learning language processing model and the AI agent may be trained to, for example, provide follow-up questions regarding the particular visualization attribute, treatment option, or any other input provided by the medical professional. That way, the machine learning language processing model may have a pseudo conversation with the medical professional until the machine learning language processing model identifies the attribute and enough information to generate (or instruct others to generate) the visualization. That is, the interaction between the machine learning language processing model and the medical professional may take into account the history of the interaction. This may provide a capacity for a pseudo conversation that takes interaction history into account when asking follow-up questions.

In some embodiments, the analytics server may receive an input indicating an identifier (e.g., a name) of the patient via the user interface or the platform provided. Responsive to receiving the input, the analytics server can retrieve patient data (e.g., one or more patient attributes) regarding the patient from non-transitory memory containing database records of the patient database. Examples of patient attributes the analytics server may retrieve include computed tomography (CT) scans of the patient or a tumor of the patient, images of the patient or a tumor of the patient, previously collected patient attributes of the patient, such as data collected from previous health tests, among others. The analytics server may also query a treatment planner and receive a treatment plan (or proposed treatment plan) for the identified patient.

Subsequently, the AI agent may present a series of questions asking (in natural language) the visualization attributes. For instance, the analytics server can present the retrieved patient data on the user interface in another widget or a portion of the user interface separate or adjacent to the interaction interface. In some cases, the portion or widget of the user interface, including the retrieved patient data, can be separated from the interaction interface on the user interface by a line (e.g., a vertical line going across the width or length of the user interface (e.g., along the x-axis or the y-axis) of the user interface). The analytics server may then present a question such as "How do you like the treatment plan to be visualized?" and await input from the medical professional.

The visualization attribute may include any desired method of visualizing the treatment attribute as it relates to the patient. A non-limiting example of a visualization may include a DVH.

At step **206**, the analytics server may execute a machine-learning language processing model using one or more visualization attributes to generate machine-readable code instructing a visualization software module to generate a visualization corresponding to the one or more visualization attributes.

The machine-learning language processing model may be specially trained to ingest patient data/attributes along with one or more visualization attributes and generate valid computer or machine-readable code tailored for a visualization engine or software module, such that the visualization engine can generate the desired visualization using patent data.

Prior to executing the machine-learning language processing model, the analytics server may generate or train the machine learning language processing model. The analytics server can generate or train the machine learning language processing model using supervised learning, unsupervised learning, or semi-supervised learning techniques.

The analytics server may first generate a training dataset corresponding to previously treated patients and/or test patients. Accordingly, the training data set may include different sentences or paragraphs of text that correspond to the radiotherapy treatment of patients, treatment visualizations, and the corresponding code. In some embodiments, the training data may be divided into four categories: (1) patient data, (2) treatment data, (3) treatment visualization and its attributes, and (4) code associated with the visualization. As a result, the machine learning language processing model may learn how to correlate a visualization of a treatment attribute to its underlying code. Moreover, the machine learning language processing model may learn how to correlate different visualization attributes to the corresponding visualization code.

The underlying code may also include pertinent libraries used by one or more visualization engines to generate the visualization (also included within the training dataset). Different libraries may contain standardized coding language and references to generate the visualization. In one example, the code itself may refer to different libraries where the libraries can be queried by the visualization engine to generate the desired visualizations.

In some embodiments, the analytics server may train the machine-learning language processing model using the training dataset. The machine-learning language processing model may ingest the training dataset and learn how to correlate visualization attributes to the underlying code. The purpose of this training is to learn how to generate machine-readable code that would generate a visualization with visualization attributes. For instance, the machine-learning language processing model may ingest a set of visualization attributes and then predict a visualization that corresponds to the visualization attributes. The machine-learning language processing model may then predict the underlying code and the libraries to generate that predicted visualization.

In a non-limiting example, the user may input, "Let me see how doses affect different organs if we go with this treatment plan." The machine-learning language processing model may then determine that the visualization attributes indicate that the user is interested in viewing a DVH of the patient's organs (both OARs and PTVs). The machine-learning language processing model will then determine one or more, or a set of libraries to generate the DVH table and underlying code for a visualization engine to generate the DVH.

In some embodiments, the analytics server may use a general-purpose machine-learning language processing model and utilize an in-context learning paradigm. In-context learning may refer to the model's ability to adapt and generate responses based on the immediate context of a given interaction. In this approach, the model may act without relying on external memory or updates to its parameters. In this approach, the model may utilize the provided prompt (including the visualization attributes) to infer the medical professional's intent. For instance, the analytics server may augment the model's prompt (containing the user request and the visualization attributes) with instructions regarding how to analyze the standardized 2D, 3D, or 4D data (such as providing information on the data format or explaining interface to some generic data manipulation library).

Additionally, or alternatively, the analytics server may (e.g. further) train a specially trained version of the machine-learning language processing model by using a transfer learning technique. For instance, the analytics server may utilize a general-purpose machine-learning language processing model and continuously optimize the model's network parameters using a specialized training set containing examples of machine-readable code doing various visualizations based on the standardized outputs, documentation, generic user wishes in visualization (e.g., conventional resolutions and/or color-conventions).

The machine-learning language processing model can then be instructed (e.g., via prompting) to either use a generic visualization protocol or a specific visualization engine that has been tailored for visualizing radiotherapy-specific graphics. In some embodiments, the interface for the visualization engine may be trained and configured for the machine-learning language processing model and the machine-readable code it predicts/produces. Moreover, when using a particular visualization engine, the machine-learning language processing model may be specially tuned using the configurations of the visualization engine. For instance, a particular visualization engine may use certain specific libraries to generate the visualization. In those embodiments, the machine-learning language processing model may be trained such that the generated code is specifically tailored for the visualization engine (e.g., trained such that the code includes code that uses the same libraries used by that particular visualization engine). The machine-readable code generated via the machine-learning language processing model may either include the actual code from a digital library or include functions that call a particular code within (or otherwise associated with) the digital library.

As used herein, a digital library may refer to one or more code libraries that include binary information, functions, commands, and/or other data needed to generate the visualizations discussed herein. In some embodiments, the data associated with the digital library may be installed in the visualization engine and the functions and commands that use the digital library may be included in the code that is transferred to the visualization engine. For instance, when the machine-learning language processing model generates machine-readable code, it may use commands and functions that are found in a certain digital visualization library. However, the actual compilation of the code may be performed in the software module (e.g., the visualization engine). Therefore, machine-readable code may merely include functions/commands that call particular functions within a digital library.

Additionally, or alternatively, the machine-learning language processing model may also include the actual code (e.g., pieces of binary code that correspond to a particular digital library) within the sent machined readable code.

In some embodiments, the user request (inputted by the medical professional) can be augmented using a template description of typical things that benefit from visualization. For instance, a template used to augment the request received via the medical professional may indicate that there is a need to include the ISO center in the visualization (though the medical professional did not specifically include that instruction). The template may also include code or other instructions regarding how to achieve the additional features (e.g., a digital library that can be used to include the ISO center).

At step 208, the analytics server may transmit the machine-readable code to the software module. The code predicted/generated by the machine-learning language processing model may include executable instructions that can be parsed and compiled via the visualization engine. The code predicted by the machine-learning language processing model may also include a list of references and libraries to generate the requested visualization. Accordingly, the code predicted by the machine-learning language processing model may include instructions on how to generate the visualizations, attributes describing the visualization, and/or indexed data to generate the visualization.

In step **210**, in response to receiving the visualization from the visualization software module, the analytics server may present the visualization via the user interface.

After transmitting the code predicted by the machine-learning language processing model, the visualization engine may receive the code generated/predicted by the machine-learning language processing model and generate the requested visualization. The visualization engine may then transmit the visualization back to the analytics server. At that point, the analytics server may dynamically populate a portion of the user interface using the received visualization.

After presenting the visualization, the analytics server may provide an additional input element on the user interface. Using the additional input element, the medical professional may provide an input corresponding to the quality of the visualization. That is, the medical professional may provide input regarding whether the visualization presented matches the visualization that was requested earlier (e.g., does the visualization have attributes corresponding to the original input of the medical professional in Step 202). The analytics server may then re-train or re-calibrate the machine-learning language processing model using the input received from the medical professional.

In some embodiments, the analytics server may interact with the medical professional via the interaction interface. In this way, the medical professional may request revisions to the presented visualization (e.g., the medical professional may input instructions to add a new element to the presented visualization or remove or change an existing element). The analytics server may then revise the visualization accordingly (by executing the machine-learning language processing model using the new input, generating new code, transmitting the code to the visualization engine, and presenting the new visualization). The analytics server may then re-train or re-calibrate the machine-learning language processing model using the additional inputs and revision instructions received from the medical professional.

In some embodiments, the analytics server may store a log of all visualizations and revisions (if applicable) associated with different medical professionals, clinics, and/or patients. In this way, the medical professional or an administrator can later refer to one of the previous visualizations for reviewing purposes and/or approval.

In some embodiments, the user may adjust the radiotherapy treatment plan in accordance with the user's input (e.g., an input or a visualization change). For example, the user viewing the radiotherapy treatment plan at the user interface can provide an input (e.g., a text input or a selection of a button) at the user interface that the radiotherapy treatment plan is incorrect. Responsive to the input indicating the radiotherapy treatment plan is incorrect, the analytics server can execute the machine learning language processing model using the input or an indication of the input that the radiotherapy treatment plan is incorrect to generate a second visualization where at least one attribute is revised. In a non-limiting example, a medical professional may input, "This DVH does not seem right. Let's lower the OAR dosage to 5 Gy." As a result, the analytics server may instruct the plan optimizer computer model to generate a new RTTP using the new attribute (e.g., 5 Gy threshold) and display the results.

In an example, a physician decides to prescribe a new treatment for a patient. Before choosing between two different treatment plan alternatives, the physician desires to check how the patient's weight fares in comparison to other treated patients. The treatment planner may not be able to illustrate this request. However, the treatment planner may be communicating with a server and a database that may include other patient data that has been indexed based on their proper attributes (e.g., patient data and treatment plans).

The physician may access an AI-backed interaction interface discussed herein and ask (in natural language) to create a 2D graph illustrating the weight distribution of previously treated patients (sometimes filtered via attributes such as geographic location and/or age) along with the present patient. The physician may also request each patient's RTTP to be visualized (e.g., DVH of the treated patients and DVH associated with the different alternative plans). The interaction interface may use an LLM that has been trained with visualization code, e.g., Python language, regarding how to create the Python code. Using the training, the LLM may first understand the content of the physician's request and then generate Python code that can query relevant data and generate the visualization desired by the physician. For instance, the LLM may generate code that can visualize the treatment plans within the bounds identified by the physician and then code that can visualize the treatment plans in comparison with the present patient.

The LLM can turn the standardized 1D vector input into the graph requested by the physician, test itself for code correctness, get the weight data from the database as input, and transmit the code to a visualization engine. Using the code generated by the LLM, a visualization engine can generate the graph requested by the physician. Using the graph of the patient data in comparison to other similar patients in the same weight class, the physician may make an informed decision.

In another example, a treatment planner, following a specified protocol, aims to assess the permissible dose intensity on a bowel structure near the treatment target, considering the patient's previous cancer treatment. For instance, the planner generates a treatment plan for a patient and inputs, "Show me how hot the dose is in a bowel structure near the tumor." Using the methods and systems discussed herein, the machine-learning language processing model may generate the code enabling a visualization engine to generate a 3D visualization of the patient's past dose distribution relative to the bowel structure, along with a calculated average dose to the bowel.

The code generated by the machine-learning language processing model may enable (by generating code) the analytics server, the planner, and/or the visualization engine to transform standardized 4D and 3D structural data into a color-coded 3D voxel graph displaying the dose distribution while also calculating the mean dose. The machine-learning language processing model may also self-verify the code's accuracy, retrieve the dose data from the database, and present an interactive graph. This visualization may allow the planner/physician to manipulate and examine the voxel data by rotating and slicing through various aspects, facilitating informed adjustments to the bowel's treatment objectives in the current plan.

The methods and systems used herein can be used to visually compare patient attributes during their treatment. For instance, a patient may be scheduled to receive several treatments (radiation doses). Using the methods and systems discussed herein, a physician can visually inspect the patient's progress by comparing various attributes at different times during the different stages of treatment.

In another example, a patient has received at least one fraction of their treatment and is scheduled to receive a subsequent fraction of their treatment. New medical images of the PTV and OAR are prepared and compared with the original medical images (from which the treatment plan has been generated) to identify/evaluate the patient's progress. A treatment planner computer model is used to generate a new treatment using the latest images. However, the treatment planning model may detect that a pre-set tolerance variable (e.g., dose limit) has been exceeded in a certain organ.

A physician may access the interaction interface and request the creation of a 2D graph showing the dose values from previous fractions for the patient together with the new value along with the tolerance value. The machine-learning language processing model may predict code that can enable the analytics server and/or the visualization engine to turn standardized 2D data input into a 2D graph. Using this visualization, the physician may determine whether to change the plan to account for this dose value "problem" or continue treating with the existing plan.

In a non-limiting example, a treatment planner may access a platform having an input element (e.g., the platform discussed herein) and may input their request in plain English (or other languages). Specifically, the treatment planner may input "show me a DVH comparison between this plan and plan X." In this example, Plan X may refer to a previous version of the patient's treatment plan and/or a treatment plan generated via a different model. As a result, the analytics server may use the LLM discussed herein to generate machine-readable code that can search the database for other treatment plans for the same patient. The machine-readable code may then cause a visualization that compares DVHs from the current plan and plan X.

As a result, the analytics server may display the GUI **300** that includes DVH curves associated with the current plan and plan X. Specifically, the GUI **300** may include various lines that represent a difference between the current plan and plan X. For instance, the GUI **300** includes solid and dashed lines where the solid lines (e.g., line **302**) represent a DVH curve of a particular anatomical region or organ using the current plan and the dashed lines (e.g., line **304**) represent a DVH curve for the same organ using plan X.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means, including memory sharing, message passing, token passing, network transmission, etc.

The actual software code or specialized control hardware used to implement these systems and methods is not limited to the claimed features or this disclosure. Thus, the operation and behavior of the systems and methods were described without reference to the specific software code, and it is understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate the transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disc, and Blu-ray disc, where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A method comprising:
presenting, by a processor on a user device, a user interface comprising an interaction interface for a medical professional operating the user device;
receiving, by the processor from the interaction interface, one or more visualization attributes associated with a radiation therapy treatment of a patient;
executing, by the processor, a machine-learning language processing model using the one or more visualization attributes to generate machine readable code instructing a visualization software module to generate a visualization corresponding to the one or more visualization attributes;
transmitting, by the processor, the machine-readable code, to the software module; and
in response to receiving the visualization from the visualization software module, presenting, by the processor, the visualization via the user interface.

2. The method of claim 1, wherein the machine-readable code comprises a digital library to generate the visualization.

3. The method of claim 1 or claim 2, wherein the machine-learning language processing model is specifically trained for a plan optimizer that generates the radiation therapy treatment of the patient.

4. The method of any preceding claim, wherein the machine-learning language processing model is specifically trained for the visualization using at least one configuration of the visualization engine.

5. The method of any preceding claim, further comprising:
recalibrating, by the processor, the machine-learning language processing model using an input corresponding to a quality of the visualization.

6. The method of any preceding claim, further comprising:
adjusting, by the processor, an attribute of the treatment plan in accordance with an input received after presenting the visualization.

7. The method of any preceding claim, wherein the visualization is a comparison between an attribute of the treatment plan at two different times.

8. A system comprising:
a non-transitory computer readable medium having instructions that when executed, cause a processor to:
present, on a user device, a user interface comprising an interaction interface for a medical professional operating the user device;
receive, from the interaction interface, one or more visualization attributes associated with a radiation therapy treatment of a patient;
execute a machine-learning language processing model using the one or more visualization attributes to generate machine readable code instructing a visualization software module to generate a visualization corresponding to the one or more visualization attributes;
transmit the machine-readable code, to the software module; and
in response to receiving the visualization from the visualization software module, present the visualization via the user interface.

9. The system of claim 8, wherein the machine-readable code comprises a digital library to generate the visualization.

10. The system of claim 8 or claim 9, wherein the machine-learning language processing model is specifically trained for a plan optimizer that generates the radiation therapy treatment of the patient.

11. The system of any of claim 8 to claim 10, wherein the machine-learning language processing model is specifically trained for the visualization using at least one configuration of the visualization engine.

12. The system of any of claim 8 to claim 11, wherein the instructions further cause the processor to recalibrate the machine-learning language processing model using an input corresponding to a quality of the visualization.

13. The system of any of claim 8 to claim 12, wherein the instructions further cause the processor to adjust an attribute of the treatment plan in accordance with an input received after presenting the visualization.

14. The system of any of claim 8 to claim 13, wherein the visualization is a comparison between an attribute of the treatment plan at two different times.

15. The system of any of claim 8 to claim 14, comprising:
the machine-learning language processing model; and
a processor in communication with the machine-learning language processing model.
